# EUROPEAN PATENT APPLICATION

(11) **EP 4 742 254 A1**
(43) Date of publication of application: **13.05.2026**
(21) Application number: 25205908.4
(22) Date of filing: 30.09.2025
(51) Int. Cl.: G16C 60/00, G16C 20/30, G16C 20/70

(54) **SYSTEM, DEVICE AND METHOD FOR MATERIAL SUBGRAPH MODEL**

(30) Priority: 12.11.2024 US 202463719527 P; 17.04.2025 US 202519182427
(71) Applicant: Samsung Display Co., Ltd., Yongin-si, Gyeonggi-do 17113 (KR)
(72) Inventor: QU, Shuhui, San Jose, 95134 (US); PARK, Cheol Woo, San Jose, 95134 (US); CHENG, Qisen, San Jose, 95134 (US); LEE, Janghwan, San Jose, 95134 (US)
(74) Representative: Marks & Clerk LLP

(57) **Abstract**

A system and a method are disclosed for material property prediction using subgraph modeling. A method may utilize artificial intelligence (Al)-based material subgraph models, including the decomposition of the molecular structure of materials, to generate graphs of subgraphs, and implement subgraph modeling to scale AI-based models for use with large and/or complex molecules and enhance the material property predictions. Aspects can involve utilizing the enhanced material property predictions to improve the overall performance of display related products. For instance, by using material property predictions with greater accuracy from subgraph modeling, materials deemed most suitable and/or efficient may be used to manufacture a display device. The method may include generating subgraphs comprising a molecular substructure of a material, and applying an AI-based model to the subgraphs to generate a material property prediction based on the molecular substructure of the material.

## Description

### BACKGROUND

### 1. FIELD

Aspects of some embodiments of the present disclosure generally relate to machine learning and/or artificial intelligence. More particularly, the subject matter disclosed herein relates to determining material properties for display related products based on artificial intelligence.

### 2. DESCRIPTION OF THE RELATED ART

Material property prediction may involve estimating the physical, chemical, mechanical, and/or optical properties of materials that can be used in display related products. Therefore, it may be desirable to utilize computational models, for example artificial intelligence-based models, to generate material property predictions that can then be utilized in various aspects of the design and/or manufacture of display related products, including material selection, performance optimization, and innovation in display technology.

The above information disclosed in this Background section is for enhancement of understanding of the background of the present disclosure, and therefore, it may contain information that does not constitute prior art.

### TECHNICAL FIELD

The disclosure generally relates to electronic devices. More particularly, the subject matter disclosed herein relates to determining material properties for display related electronic devices based on artificial intelligence.

### SUMMARY

Aspects of some embodiments of the present disclosure relate to material property prediction and/or analysis. For example, aspects of some embodiments of the present disclosure relate to improvements to the accuracy and efficiency of material property predictions by utilizing artificial intelligence and/or material subgraph models.

Material property prediction may involve estimating the physical, chemical, mechanical, and/or optical properties of materials that can be used in display related products. Therefore, it may be desirable to utilize computational models, for example artificial intelligence-based models, to generate material property predictions that can then be utilized in various aspects of the design and/or manufacture of display related products, including material selection, performance optimization, and innovation in display technology. However, there may be issues associated with applying artificial intelligence (AI) techniques to material property prediction, in a manner that maintains the robustness and/or efficiency of AI and can scale to the relatively large size and complexity of the molecular structure of materials that may be used for display related products.

Aspects of some embodiments of the present disclosure relate to systems and methods for AI-based material subgraph models, including the decomposition of the molecular structure of materials, generating graphs of subgraphs, and implementing subgraph modeling in a manner that may scale AI-based models to large and/or complex molecules and enhances their expressive power. Thus, the disclosed embodiments may improve the overall performance of display related products by utilizing materials deemed most suitable and/or efficient.

In some embodiments, a method includes: generating, by a processor, subgraphs, each of the subgraphs including a molecular substructure of a material; applying, by the processor, an AI-based model to the subgraphs to generate a material property prediction based on the molecular substructure of the material; determining, by the processor, a function related to the material for production of a target device based on the material property prediction; and transmitting, by the processor, a signal to a component to control the component to execute the function related to the material for the production of the target device.

In some embodiments, the method may further include decomposing a graph of a molecular structure of the material into the molecular substructures.

In some embodiments, the decomposing may include Breaking Retrosynthetically Interesting Chemical bonds (BRIC) decomposition.

In some embodiments, the method may further include generating embeddings of the subgraphs based on the subgraphs.

In some embodiments, generating the embeddings of the subgraphs may include processing the subgraphs by a graph neural network.

In some embodiments, the method may further include generating a graph of subgraphs based on the embeddings of the subgraphs.

In some embodiments, the graph of subgraphs may include nodes and edges.

In some embodiments, each of the nodes correspond to one of the subgraphs and a value for each of the nodes correspond to one of the embeddings of the subgraphs.

In some embodiments, each of the edges represent a relationship between the subgraphs.

In some embodiments, the method may further include generating updated node embeddings based on the graph of subgraphs.

In some embodiments, generating the updated node embeddings may include processing the graph of subgraphs by a graph neural network.

In some embodiments, the AI-based model analyzes the graph of subgraphs and models the relationships between the subgraphs.

In some embodiments, the target device comprises an organic light-emitting diode (OLED) display device.

In some embodiments, the material property prediction is based one or more of: a physical property of the material, a chemical property of the material, mechanical property of the material, or an optical property of the material.

In some embodiments, a device includes: one or more processors that are configured to perform: generating subgraphs, each of the subgraphs including a molecular substructure of a material; applying an AI-based model to the subgraphs to generate a material property prediction based on the molecular substructure of the material; determining a function related to the material for production of a target device based on the material property prediction; and transmitting a signal to a component to control the component to execute the function related to the material for the production of the target device.

In some embodiments, the one or more processors are further configured to perform decomposing a graph of a molecular structure of the material into the molecular substructures.

In some embodiments, the one or more processors are further configured to perform generating embeddings of the subgraphs based on the subgraphs.

In some embodiments, the one or more processors are further configured to perform generating a graph of subgraphs based on the embeddings of the subgraphs.

In some embodiments, the one or more processors are further configured to perform analyzing the graph of subgraphs using the AI-based model and modeling relationships between the subgraphs.

In some embodiments, a system includes a processing circuit; and a memory storing instructions, which, based on being executed by the processing circuit, cause the processing circuit to perform: generating subgraphs, each of the subgraphs comprising a molecular substructure of a material; applying an AI-based model to the subgraphs to generate a material property prediction based on the molecular substructure of the material; determining a function related to the material for production of a target device based on the material property prediction; and transmitting a signal to a component to control the component to execute the function related to the material for the production of the target device.

At least some of the above and other features of the invention are set out in the claims.

### BRIEF DESCRIPTION OF THE DRAWING

In the following section, the aspects of the subject matter disclosed herein will be described with reference to example embodiments illustrated in the figures.
FIG. 1 is a block diagram depicting a system (e.g., a factory) for producing products (e.g., electronic devices, such as organic light-emitting diode (OLED) display devices) utilizing AI-based material subgraph models, according to some embodiments of the present disclosure.
FIG. 2 is a block diagram depicting a computer device for material property prediction, including an example material subgraph modeling circuit, according to some embodiments of the present disclosure.
FIG. 3 is a block diagram depicting a computer device for material property prediction, including another example of a material subgraph modeling circuit, according to some embodiments of the present disclosure.
FIG. 4 is a diagram depicting an example process for decomposing a molecular structure of a material implemented by the material subgraph modeling circuit of FIG. 2, according to some embodiments of the present disclosure.
FIG. 5 is a diagram depicting an example process for generating embeddings of subgraphs implemented by the subgraph modeling circuit of FIG. 2, according to some embodiments of the present disclosure.
FIG. 6 is a diagram depicting an example process for generating a graph of subgraphs implemented by the subgraph modeling circuit of FIG. 2, according to some embodiments of the present disclosure.
FIG. 7A is a diagram depicting an example process for generating updated embeddings of subgraphs implemented by the subgraph modeling circuit of FIG. 3, according to some embodiments of the present disclosure.
FIG. 7B is a diagram depicting an example configuration of a transformer generating updated embeddings of subgraphs and implemented by the subgraph modeling circuit of FIG. 3, according to some embodiments of the present disclosure.
FIG. 8 is a block diagram of an electronic device in a network environment 800, according to some embodiments of the present disclosure.
FIG. 9 is a flow chart depicting example operations of a method for utilizing AI-based material subgraph models and material property prediction, according to some embodiments of the present disclosure.

### DETAILED DESCRIPTION

In the following detailed description, numerous specific details are set forth in order to provide a thorough understanding of the disclosure. It will be understood, however, by those skilled in the art that the disclosed aspects may be practiced without these specific details. In other instances, well-known methods, procedures, components and circuits have not been described in detail to not obscure the subject matter disclosed herein.

Reference throughout this specification to "one embodiment" or "an embodiment" means that a particular feature, structure, or characteristic described in connection with the embodiment may be included in at least one embodiment disclosed herein. Thus, the appearances of the phrases "in one embodiment" or "in an embodiment" or "according to one embodiment" (or other phrases having similar import) in various places throughout this specification may not necessarily all be referring to the same embodiment. Furthermore, the particular features, structures or characteristics may be combined in any suitable manner in some embodiments (e.g., in one or more embodiments). In this regard, as used herein, the word "exemplary" means "serving as an example, instance, or illustration." Any embodiment described herein as "exemplary" is not to be construed as necessarily preferred or advantageous over other embodiments. Additionally, the particular features, structures, or characteristics may be combined in any suitable manner in one or more embodiments. Also, depending on the context of discussion herein, a singular term may include the corresponding plural forms and a plural term may include the corresponding singular form. Similarly, a hyphenated term (e.g., "two-dimensional," "pre-determined," "pixel-specific," etc.) may be occasionally interchangeably used with a corresponding non-hyphenated version (e.g., "two dimensional," "predetermined," "pixel specific," etc.), and a capitalized entry (e.g., "Counter Clock," "Row Select," "PIXOUT," etc.) may be interchangeably used with a corresponding non-capitalized version (e.g., "counter clock," "row select," "pixout," etc.). Such occasional interchangeable uses shall not be considered inconsistent with each other.

Also, depending on the context of discussion herein, a singular term may include the corresponding plural forms and a plural term may include the corresponding singular form. It is further noted that various figures (including component diagrams) shown and discussed herein are for illustrative purpose only, and are not drawn to scale. For example, the dimensions of some of the elements may be exaggerated relative to other elements for clarity. Further, if considered appropriate, reference numerals have been repeated among the figures to indicate corresponding and/or analogous elements.

The terminology used herein is for the purpose of describing some example embodiments only and is not intended to be limiting of the claimed subject matter. As used herein, the singular forms "a," "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

It will be understood that when an element or layer is referred to as being on, "connected to" or "coupled to" another element or layer, it can be directly on, connected or coupled to the other element or layer or intervening elements or layers may be present. In contrast, when an element is referred to as being "directly on," "directly connected to" or "directly coupled to" another element or layer, there are no intervening elements or layers present. Like numerals refer to like elements throughout. As used herein, the term "and/or" includes any and all combinations of one or more of the associated listed items.

The terms "first," "second," etc., as used herein, are used as labels for nouns that they precede, and do not imply any type of ordering (e.g., spatial, temporal, logical, etc.) unless explicitly defined as such. Furthermore, the same reference numerals may be used across two or more figures to refer to parts, components, blocks, circuits, units, or modules having the same or similar functionality. Such usage is, however, for simplicity of illustration and ease of discussion only; it does not imply that the construction or architectural details of such components or units are the same across all embodiments or such commonly-referenced parts/modules are the only way to implement some of the example embodiments disclosed herein.

Unless otherwise defined, all terms (including technical and scientific terms) used herein have the same meaning as commonly understood by one of ordinary skill in the art to which this subject matter belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

As used herein, the term "module" refers to any combination of software, firmware and/or hardware configured to provide the functionality described herein in connection with a module. For example, software may be embodied as a software package, code and/or instruction set or instructions, and the term "hardware," as used in any implementation described herein, may include, for example, singly or in any combination, an assembly, hardwired circuitry, programmable circuitry, state machine circuitry, and/or firmware that stores instructions executed by programmable circuitry. The modules may, collectively or individually, be embodied as circuitry that forms part of a larger system, for example, but not limited to, an integrated circuit (IC), system on-a-chip (SoC), an assembly, and so forth.

In recent years, the display industry has been focused on developing cutting-edge, next-generation display materials for products. The aim may be to enable new types of high-efficiency and low-cost display related products. A critical process to realizing improved products, may be obtaining material properties efficiently and accurately. Efficient determination of the properties and/or characteristics of potential materials to be used for fabricating products may accelerate the development and optimization of new materials.

Density Functional Theory (DFT) simulations may be used to obtain the material properties based on physical constraints before any experimental validation. However, the accuracy and/or reliability of the predictions of material properties provided by DFT simulations (and other conventional mechanisms) may be limited. For example, DFT approaches may experience delays (relatively long time periods to reach steady-state solutions). The computational inefficiency of some current material property analysis mechanisms, such as DFT, may limit their application in the rapid discovery of new materials.

Additionally, there may be some AI-based approaches currently utilized to provide automated material property analysis and/or prediction tools. Nonetheless, these AI-based models may not effectively capture the various relationships that may be associated with the complex molecular structure of materials, resulting in a loss of crucial relational information.

These AI-based models may not be suitable for capturing long-range interactions, which may be a limitation that can reduces the model's expressiveness, leading to lower performance when analyzing large molecules. Furthermore, the sampling approaches for these AI-based models may rely on conventional modeling functions (e.g., node and/or edge removal) which may not be suitable for leveraging chemical decomposition techniques, and thereby may reduce the model's ability to efficiently handle large molecules (e.g., molecules with hundreds of atoms), where many materials used for display-related devices may include large molecules. In some cases, limitations in material property analysis and/or prediction may arise from a dependence on publicly available datasets, which may contain relatively small molecules (e.g., consisting of only tens of atoms).

To improve material property analysis and/or predictions, and in turn, enhance display-related products, the embodiments implement functions related to generating, training, and utilizing AI-based subgraph models that may scale to large and complex molecules and enhance expressive power to improve accuracy and/or performance of material property predictions. As alluded to above, material utilized for producing display-related devices may often involve large molecules, and thus may require AI-based models that can handle such complexity. The AI-based subgraph models, as disclosed herein, are implemented to scale to large molecules by leveraging subgraphs. For example, the AI-based subgraph models may leverage subgraph decomposition for material (e.g., chemical) molecular structure instead of relying on more conventional AI modeling techniques (e.g., edge deletion, node deletion, etc.)

The AI-based subgraph models, as disclosed herein, may also achieve enhanced expressive power. By enhancing the expressive power (e.g., effectively modeling long-range interactions through the relational modeling between subgraphs) of the AI-based subgraph models, these models may achieve better performance in predicting material properties.

Aspects of some embodiments of the present disclosure provide for AI-based subgraph models to mitigate (e.g., to overcome) the aforementioned issues by implementing functions that may include: subgraph selection, which may involve utilizing a domain-specific subgraph sampler to identify and/or select meaningful substructures within a molecular graph based on specialized chemical principles(e.g., Breaking Retrosynthetically Interesting Chemical bonds (BRIC), hierarchical decomposition strategies, etc.); subgraph representation, which may include representing each subgraph as a corresponding node within a constructed graph (referred to herein as a graph of subgraphs), where edges between nodes may denote interactions or relationships between the corresponding subgraphs; interaction modeling, which may involve capturing and/or modeling the interactions (and dependencies) between the subgraphs within the graph of subgraphs to preserve relational information and enable the representation of complex structural patterns; and material property prediction, which may involve applying a neural network to the graph of subgraphs to predict material properties based on the aggregated and interacted subgraph representations.

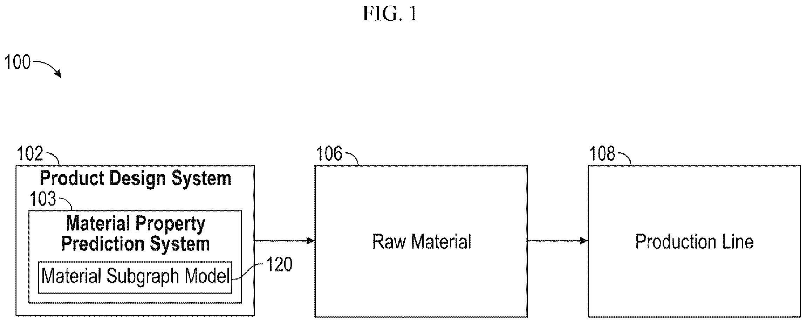

FIG. 1 is a block diagram depicting a system 100 (e.g., a factory) for producing display-related products (e.g., electronic devices, such as organic light-emitting diode (OLED) display devices), according to some embodiments of the present disclosure.

Manufacturing products (e.g., in a factory or a production line) may include various processes to ensure certain quality standards are satisfied. In some embodiments, the system 100 (e.g., the factory) of FIG. 1 may produce products (e.g., electronic devices, such as display devices, integrated circuits, and/or the like), which may be referred to as target devices in the present disclosure. As seen in FIG. 1, the system 100 may include a production line 108. The production line 108 may include machines, machinery, and/or devices that take raw materials 106 and/or components as inputs and assembles, constructs, and/or produces one or more products, such as the display devices (e.g., OLED display devices).

In manufacturing target devices such as some display-related products (e.g., OLED display devices), material property predictions may be used to identify and select the most suitable organic materials for each layer of the target device. The production design system 102 may include a material property prediction system 103, which may be implemented as a computer device having the capability to generate, train, and/or utilize a material subgraph model 120. The material subgraph model 120 may be an artificial intelligence (AI) and/or machine learning (ML) based model (also referred to herein as "neural networks") that can be generated, trained, and/or utilized in accordance with the subgraph modeling functions, as disclosed herein, and may realize improved efficiency and/or accuracy in predicting material properties. The production design system 102 may then leverage the enhanced accuracy and/or prediction performance of the material property prediction system 103 for its functions relating to the design, testing, and/or production of display related products.

The material subgraph model 120 may be used to implement computational modeling techniques, which can be used to generate material property predictions based on the physical, chemical, electrical, mechanical, and/or optical properties of materials (e.g., the molecular structure of potential material). Subsequently, based on the enhanced material property predictions implemented by the material property prediction system 103 and/or the material subgraph model 120, the production design system 102 may be able to perform a plurality of functions (e.g., select, design, fabricate, validate, and/or the like) involving one or more of the raw materials 106 that may be deemed suitable and/or optimal to design (e.g., computer aided design) the product, which may ultimately be used for the actual manufacture of the product in the production line 108.

The product design system 102 may have the capability to perform aspects related to automated simulation, design, fabrication and/or validation of the raw materials 106 such as one or more different organic material for each layer of the OLED display device. As an operational example, the product design system 102 may utilize the material subgraph model 120 in a computational prediction to determine and/or analyze key characteristics for the raw materials 106 such as light emission efficiency, stability, and charge transport properties prior to subsequent synthesis, testing, and/or utilization (e.g., for manufacturing products) of the raw materials 106. For example, the product design system 102 may utilize enhanced material property predictions (e.g., generated by the material property prediction system 103 and/or material subgraph model 120) to select (e.g., molecules that may meet determined criteria for raw materials 106) and/or filter (e.g., molecules that may not meet determined criteria for raw materials 106) the raw materials 106 and/or molecular structure of raw materials 106 for subsequent designing, fabrication, validation, and/or utilization of raw materials 106.

In another example, the product design system 102 may control and/or execute functions involved in synthesizing the raw materials 106. For instance, the product design system 102 may control one or more automated functions for a chemical reactor to synthesize raw materials 106 using the molecules selected based on the enhanced material property predictions (e.g., generated by the material property prediction system 103 and/or material subgraph model 120). The product design system 102 may control and/or execute a plurality of functions involved in material fabrication and/or synthesis for raw materials 106. Thus, enhanced material property prediction may be leveraged in fabrication and/or synthesis of raw materials 106 to increase the speed, efficiency, and performance of the process (e.g., improving the selected candidate molecules and/or materials, etc.) which may reduce the overall time consumed (e.g., delay between molecular design to validation of raw materials 106) and may optimize the design and/or performance of synthesized raw materials 106.

In another operational example, the product design system 102 may control and/or execute functions involved in automated testing and/or validation of the raw materials 106 based on the enhanced material property predictions (e.g., generated by the material property prediction system 103 and/or material subgraph model 120). For instance, the product design system 102 may control an automated test of synthesized raw materials 106 (e.g., OLED test materials), which may involve analyzing the emitted light from products using the raw materials 106 to measure the operational properties (e.g., electroluminescence) thereby validating and/or confirming (e.g., comparing measured properties of testing against theoretical and/or predetermined properties) the performance of the raw materials 106. Thus, enhanced material property prediction may be leveraged in validation and/or testing of raw materials 106 to ensure that the fabricated and/or synthesized raw materials 106 (and products manufactured using the raw materials 106) meet desired quality and/or criteria (e.g., color, luminance, and/or the like) prior to being utilized downline in the system 100, for instance in the production line 108 for manufacturing of display-related products (e.g., synthesized materials are processed into films for layers).

In another operational example, the product design system 102 may control and/or execute functions downline in the system 100, for instance in the production line 108, involved in manufacture of the display-related devices after testing and/or validating raw materials 106. For example, the product design system 102 may communicate (e.g., transmit) a command (e.g., control signal) to a system in the production line 108, such as a system of manufacturing and/or fabrication machines, to control executing an automated production of the OLED display device using the selected material (e.g., obtaining the selected material from raw materials 106) based on the enhanced material property predictions (e.g., generated by the material property prediction system 103 and/or material subgraph model 120), thus optimizing the overall display performance and minimizing development time and cost.

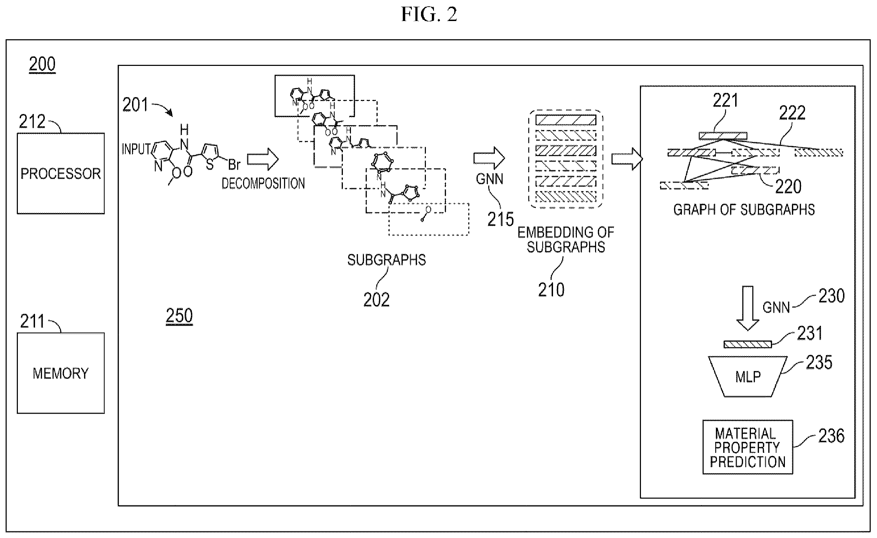

FIG. 2 is a block diagram depicting a computer device 200 for material property prediction, including a material subgraph modeling circuit 250 implementing subgraph modeling, according to some embodiments of the present disclosure.

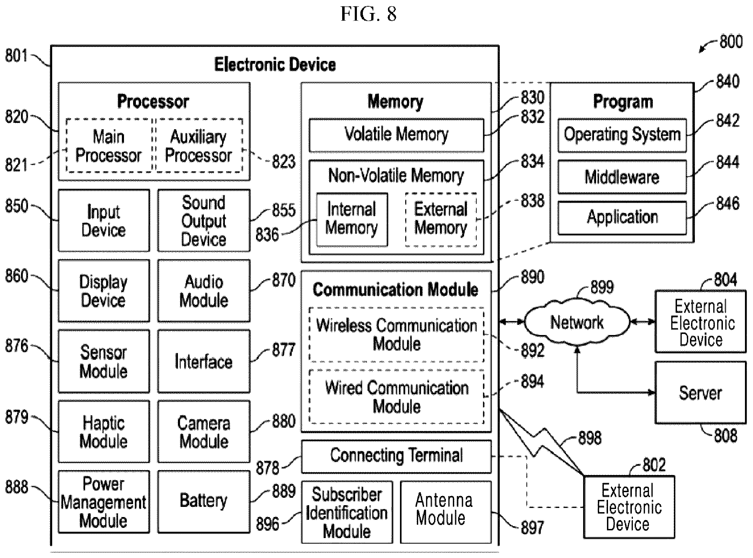

As illustrated in FIG. 2, the computer device 200 (e.g., one or more computers and/or one or more computer systems) may include a memory 211 (e.g., a memory and/or a storage), a processor 212, and a material subgraph modeling circuit 250 configured for implementing subgraph modeling, as disclosed herein. The memory 211 may correspond to the memory 830 of FIG. 8. The processor 212 may correspond to the processor 820 of FIG. 8. As a general description, the material subgraph modeling circuit 250 may execute one or more functions related to subgraph modeling, as disclosed herein, which may involve implementing material (e.g., chemical) subgraph sampling, obtaining embeddings of subgraphs, generating graphs of subgraphs, modeling interactions between subgraphs, and generating predictions of material property predictions based on the subgraphs.

According to some embodiments, the computer device 200 may utilize AI-based models (e.g., subgraph model 120) that are generated, trained, and/or utilized by the material subgraph modeling circuit 250 and related functions, thus implementing improved accuracy, efficiency, and/or performance. For example, the material subgraph modeling circuit 250 may be configured to perform one or more of the related functions, as disclosed herein, during a training phase and/or during an inference phase of the AI-based models (e.g., subgraph model 120).

The computer device 200 may include a computer system that is capable of AI-related functions including model training, computations, inference, and various AI-based applications. For example, the computer device 200 may be implemented as, for example, and without limitation, a desktop PC, a laptop, a smartphone, a tablet PC, a server, and/or the like. The computer device 200 may include a system in which a cloud computing environment is established. However, some embodiments are not limited thereto. The computer device 200 may be implemented as any system, device, or apparatus which is capable of AI-based applications and functions (e.g., material property predictions, subgraph modeling, etc.), such as a material property prediction system 103 (e.g., shown in FIG. 1), as described herein. In some embodiments, the computer device 200 may implement various functions (e.g., material selection, product design, etc.) related to manufacturing and/or inspection of an OLED display device, such as a production design system 102 (e.g., shown in FIG. 1), as disclosed herein. The computer device 200 may include one or more processors for performing one or more of the processes of the present disclosure.

In some embodiments, the memory 211 may store data and/or AI models associated with AI-based applications, as disclosed herein (e.g., material property prediction, subgraph modeling, etc.), including material subgraph model 120. In some embodiments, the memory 211 may store models generated, trained, and/or utilized by the material subgraph modeling circuit 250. In some embodiments, the memory 211 may store the material property predictions generated by the material subgraph modeling circuit 250, and utilizing subgraph modeling functions, as disclosed herein.

In some embodiments, the processor 212 may include various processing circuitry and may control overall operations of the computer device 200, including AI-based applications supported by the AI models (e.g., material subgraph model 120) generated, trained, and utilized by the material subgraph modeling circuit 250, as disclosed herein. In some embodiments, the processor 212 may include various processing circuitry (e.g., one or more processing circuits) and may control overall operations of the computer device 200 (e.g., the computer system), including AI-based applications supported by the material property predictions 236 generated by the material subgraph modeling circuit 250, as disclosed herein. In some embodiments, the processor 212 may be implemented, for example, as a digital signal processor (DSP), a microprocessor, or a time controller (TCON), or the like, but is not limited thereto. The processor 212 may, for example, and without limitation, be one or more of a dedicated processor, a central processing unit (CPU), a micro controller unit (MCU), a micro processing unit (MPU), a controller, an application processor (AP), a communication processor (CP), an ARM processor, or the like, or may be defined as one of the terms above. Also, the processor 212 may be implemented as a system on chip (SoC) in which a processing algorithm is provided, or may be implemented in a form of a field programmable gate array (FPGA), or the like, but is not limited thereto.

The material subgraph modeling circuit 250 may be implemented utilizing any suitable hardware, firmware (e.g. an application-specific integrated circuit), software, or a combination of software, firmware, and hardware. The material subgraph modeling circuit 250 may be configured to implement material subgraph sampling, as described in greater detail herein. Material subgraph sampling may utilize chemical decomposition techniques, such as BRIC, hierarchical decomposition strategies, and/or the like, to extract molecular substructures related to materials. Thus, the material subgraph modeling circuit 250 may be configured to perform the extraction of functional groups and/or structural motifs that may be critical to determining material properties.

The material subgraph modeling circuit 250 may be configured to implement graphing of subgraphs which provide a modular (e.g., having multiple segments and/or portions) and graph-based representation of properties, as described in greater detail herein. Subgraphs may involve generating a new graph where selected subgraphs may serve as nodes, and edges may represent their interactions. Thus, the material subgraph modeling circuit 250 may model long-range dependencies between subgraphs, which may support accurate material property predictions.

The material subgraph modeling circuit 250 may be configured to implement modeling of interactions between subgraphs, as described in greater detail herein. Thus, the material subgraph modeling circuit 250 may captures the dependencies and/or interactions among subgraphs by leveraging the graph-based representation of subgraphs, preserving relational information and can achieve improved accuracy in material property prediction by maintaining the relational interactions. By modeling interactions between subgraphs, the material subgraph modeling circuit 250 may provide an enhanced expressiveness level for AI models (e.g., greater than that of the 3-Weisfeiler-Lehman (3-WL) test), which can enable the AI models to distinguish complex and large-scale graph structures in a manner that may improve upon the capabilities of simpler models.

FIG. 2 illustrates that the material subgraph modeling circuit 250 may be configured to receive an input 201, which may include data representing a molecular structure of material (e.g., chemicals) related to the production and/or manufacturing of a product. In some embodiments, the input 201 may be an initial graph representation of the molecular structure of the material, where the chemical composition of the material can include large and complex molecules (e.g., having hundreds of atoms). The material subgraph modeling circuit 250 may utilize the input 201 to perform material subgraph sampling. For example, the material subgraph modeling circuit 250 may execute decomposition of the molecular structure represented in the input 201 to extract one or more molecular substructures, where the molecular substructures may further be represented as subgraphs 202. In some embodiments, the material subgraph modeling circuit 250 may be configured to implement BRIC for decomposing the input 201. The material subgraph modeling circuit 250 may perform hierarchical decomposition, in accordance with BRIC, to identify molecular substructures within molecules of the material. Decomposition may be based on a determined number (e.g., minimum, maximum, etc.) of functional groups and/or structural motifs to be extracted.

In some embodiments, the material subgraph modeling circuit 250 may be configured to control and/or determine the number of subgraphs 202 that are generated from molecular substructures as a result of the decomposition. For example, the material subgraph modeling circuit 250 may generate a determined number of subgraphs 202 such that the aggregated composition of the subgraphs 202 may cover the original graph represented by the input 201. The number of subgraphs 202 generated form decomposition may be determined based on application related factors, including but not limited to: computational resources and/or configuration of the material property prediction system 103; efficiency and/or performance related metrics (e.g., maintain the computational speed); and/or the like. In some embodiments, the material subgraph modeling circuit 250 may be configured to prioritize one or more molecular substructures to be selected for extracting during decomposition. For instance, a specific substructure of a molecule may be deemed as chemically significant to the molecular structure of a material, and thus may be prioritized by the material subgraph modeling circuit 250 over other molecular substructures with respect to being extracting during decomposition and represented in subgraphs 202. The embodiments are not limited thereto, and the material subgraph modeling circuit 250 may be configured to utilize other material and/or molecular decomposition mechanisms as deemed suitable and/or appropriate.

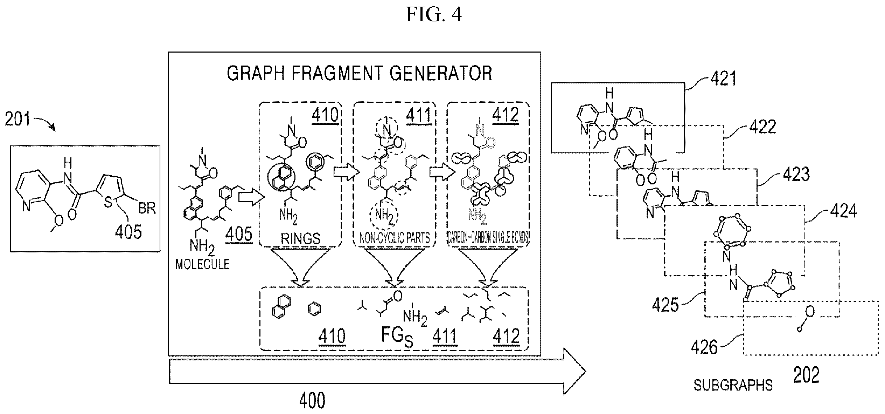

FIG. 4 depicts an example of a decomposition process 400 to generate subgraphs 202 of molecular substructures implemented by the material subgraph modeling circuit 250, according to some embodiments of the present disclosure.

In some embodiments, the material subgraph modeling circuit 250 may receive input 201 which includes data representing a structure of a molecule 405 related to the material of a product for production. For example, the molecule 405 illustrated in FIG. 4 may have a structure forming a chemical compound that includes a group of atoms (e.g., NH2, NO) bonded together. The decomposition process 400 for the molecule 405 may involve systematically fragmenting the molecule 405 by identifying parts, and/or regions (or subregions), and/or breaking bonds as deemed suitable (e.g., breaking bonds of retrosynthetic significance, etc.). The illustrated decomposition process 400 may involve decomposing the molecule 405 based on identifying rings 410, non-cyclic parts 411, and carbon-carbon single bonds 412 that comprise the molecule 405. The rings 410, non-cyclic parts 411, and carbon-carbon single bonds 412 may be extracted from the decomposition of the molecule 405 such that molecular substructures 421-426 of the molecule 405 may be formed as fragments (e.g., constructed from arranging one or more of the extracted portions of the molecule 405) that can be functional groups and/or structural motifs that are chemically pertinent for determining properties of the material. The molecular substructures 421-426 formed from decomposing the complex structure of molecule 405 may be represented as subgraphs 202. In the example of FIG. 4, each of the molecular substructures 421-426 may be represented as a different corresponding subgraph 202.

Referring again to FIG. 2, the material subgraph modeling circuit 250 may be configured to obtain subgraph embeddings (also referred to as embedding of subgraphs) 210 based on the subgraphs 202. For example, the material subgraph modeling circuit 250 may input data subgraphs 202 (representing molecular substructures) into a graph neural network (GNN) 215 to generate the subgraph embeddings 210, where each of the subgraph embeddings 210 can be a vector representation of a portion of the molecule's (input 201) properties from the corresponding subgraph 202.

The GNN 215 may implement an AI-based processing of graph-structured data, such as the subgraphs 202 including data representing molecular substructures. As an example, each subgraph 202 may be structured as a set of nodes and edges, and the GNN 215 may analyze the subgraphs 202 in order to capture features, attributes, encodings, and/or the like that relate to the data (e.g., nodes) and the relationships therebetween (e.g., edges) in the learned subgraph embeddings 210. The subgraph embeddings 210 may be obtained in a format that a machine learning model can understand and utilize for downstream tasks. Functions performed by the GNN 215 to obtain the subgraph embeddings 210 may involve tokenization, embedding, and/or positional encoding. In some embodiments, the GNN 215 may receive subgraphs 202 and obtain subgraph embeddings 210 iteratively as a function of a training process and/or during inference for a machine learning model, being executed by the processor 212.

The GNN 215 may be trained to process graph-structured data through a training process in which parameters of the GNN 215 are updated and optimized. The GNN 215 is trained based on input graphs as training data and the minimization of a loss function that quantifies the difference between predicted embeddings and target embeddings for the input graphs as training data. The parameters of the GNN 215 are iteratively updated until the GNN 215 parameters are optimized to minimize the loss function. In some examples, each input graph in the training data set includes the graph structure as represented by values for the edges and nodes representing the properties for those edges and nodes. Each input graph in the training data set can include a subgraph representing a material/molecular substructure, and for each input graph there exists a target embedding representing features, attributes, encoding and/or the like of the subgraph. The training data set upon which the GNN 215 is trained can include a dataset of material/molecular substructures as represented by subgraphs and associated target embeddings.

In some embodiments, the material subgraph modeling circuit 250 may be configured to generate a graph of subgraphs 220 as another representation of the subgraphs 202. FIG. 2 illustrates that the graph of subgraphs 220 may be a graphical structure representing data as a set of nodes 221 and edges 222, where each of the nodes 221 within the constructed graph of subgraphs 220 may represent a corresponding subgraph 202 and the edges 222 (between the nodes 221) may represent interactions and/or relationships between the corresponding subgraphs 202. Each node 221 may have associated features and/or attributes that describe its properties, and the edges 222 that connect nodes 221 may represent the relationships therebetween. The edges 222 may be directed (e.g., information flows in one direction) and/or undirected (e.g., information flows both ways) depending on the data.

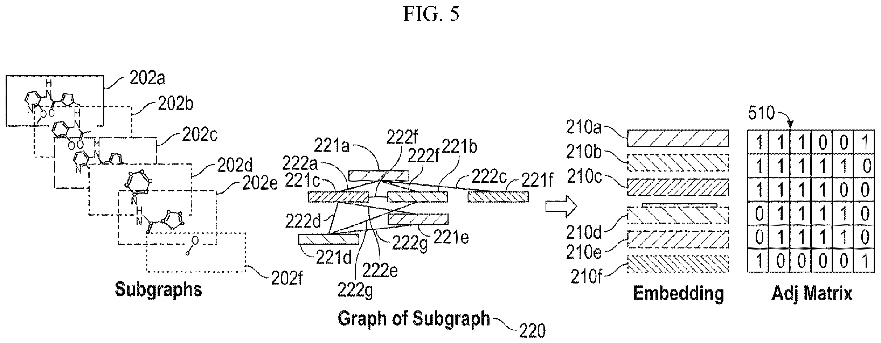

FIG. 5 depicts an example of a process for generating the graph of subgraphs 220 implemented by the material subgraph modeling circuit 250, according to some embodiments of the present disclosure.

The process in FIG. 5 may involve constructing the graph of subgraphs 220 as another graph-based representation of the subgraphs 202a-202f (including molecular substructures). As previously described, each node 221a-221f of the graph of subgraphs 220 may represent a corresponding one of the subgraphs 202a-202f, the edges 222a-222h may indicate interactions and/or relationships between the subgraphs 202a-202f, and a value for each of the nodes 221a-221f may correspond to a subgraph embedding 210a-210f (for the subgraph that the node represents). In the example graph of subgraphs 220, the node 221a may represent a subgraph 202a, and the value of node 221a may correspond to subgraph embedding 210a; the node 221b may represent a subgraph 202b, and the value of node 221b may correspond to subgraph embedding 210b; the node 221c may represent a subgraph 202c, and the value of node 221c may correspond to subgraph embedding 210c; and so on. The structure of the graph of subgraphs 220 may be represented as an adjacency matrix 510, where each cell of the adjacency matrix 510 may indicate a structural attribute of the graph of subgraphs 220 (e.g., whether an edge exists between two nodes, etc.).

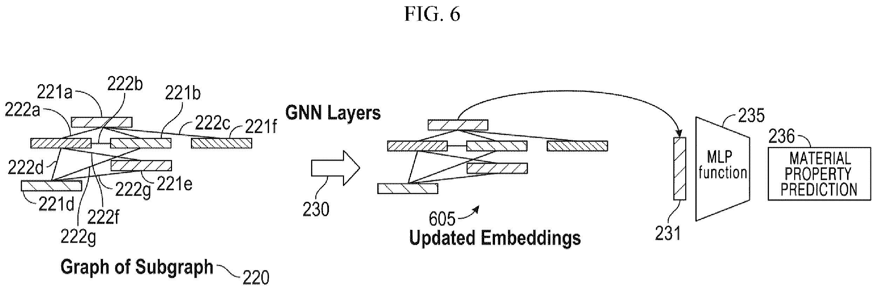

In some embodiments, the process may involve calculating the relationships and/or interactions between the subgraphs 202a-202f in a manner that can model the relationship through hierarchical relations. Distance methods, such as Jaccard distance, fingerprints, and/or the like may be used to calculate the relationships between subgraphs 202a-202f, and the relational dependencies may be graphically represented in the structure of the graph of subgraphs 220 (e.g., number of connections and/or edges, distance between nodes, etc.). For instance, the edge 222a may represent that there is a relational dependency between the node 221a (representing subgraph 202a) and the node 221c (representing subgraph 202c), and the edge 222e may represent that there is a relational dependency between the node 221c (representing subgraph 202c) and the node 221e (representing subgraph 202e). Accordingly, the process may construct the graph of subgraphs 220 such that it provides a graph-based representation that models the relationships between molecular substructures (included in the subgraphs 202a-202f). In some embodiments, interactions between molecular structures may also be modeled by a coordinate distance in the graph of subgraphs 220 when three-dimensional (3D) coordinates are provided as input. The graph of subgraphs 220 may be utilized for capturing and modeling the interactions and/or dependencies between the subgraphs 202 in a manner that can preserve relational information and enable the representation of complex structural patterns, such as the complex molecular structure of materials for products. In some embodiments, the material subgraph modeling circuit 250 may be configured to implement another graph neural network 230 in order to process the data in the graph of subgraphs 220. FIG. 6 depicts an example of a process for updating the graph of subgraphs 220 implemented by the material subgraph modeling circuit 250, according to some embodiments of the present disclosure. Accordingly, the material subgraph modeling circuit 250 may update the subgraph embeddings (represented in the graph of subgraphs 220) by utilizing the graph neural network 230.

FIG. 6 illustrates that the graph neural network 230 may utilize multiple layers to iteratively update the graph embeddings of the nodes 221 by aggregating information from neighboring nodes 221 within the graph of subgraphs 220. Thus, the material subgraph modeling circuit 250 performs a passing of information from a subgraph (e.g., a node 221) to neighboring subgraphs (e.g., a neighboring node 221). After a determined number of iterations (layers), the graph neural network 230 may generate a graph of subgraphs with updated node embeddings 605. The updated node embeddings 605 may be aggregated by the graph neural network 230, which can generate a single vector 231 (representing the aggregation of the updated node embeddings 605). In some embodiments, the embeddings of the nodes 221a-221f in the graph of subgraphs 220 that is initially constructed (e.g., prior to updates) may be utilized. In some embodiments, the final node and/or graph representation from analyzing the graph of subgraphs 220, by the graph neural network 230, may be utilized to generate the material property prediction 236 as the output. In the example process of FIG. 6, the vector 231 may be passed to a multi-layer perceptron (MLP) function 235. The MLP function 235 may be a transformation function that processes the final node and/or graph representation of the graph of subgraphs 220 (e.g., with updated node embeddings 605) for further processing tasks, such as classification, regression, and/or the like, and outputs the material property prediction 236. As previously described, the material subgraph modeling circuit 250 may model long-range interactions through the relational modeling between subgraphs in a manner that increase expressiveness (e.g., great than 3-WL), and thereby may increase the accuracy and/or performance of the material property prediction 236.

The GNN 230 may be trained to process graph-structured data through a training process in which parameters of the GNN 230 are updated and optimized. The GNN 230 is trained based on input graphs as training data and the minimization of a loss function that quantifies the difference between predicted embeddings and target embeddings for the input graphs as training data. The parameters of the GNN 230 are iteratively updated until the GNN 230 parameters are optimized to minimize the loss function. In some examples, the input graphs as training data includes the graph structure as represented by adjacency matrix and embedding values for nodes representing the subgraphs. The GNN 230 may be configured to perform message passing to propagate information across the graph by executing a plurality of message passing steps. The GNN 230 parameters may be optimized by minimizing the loss function for the predicted embeddings resulting from the message passing process as compared to the target embeddings. The training data set upon which the GNN 230 is trained can include a dataset of materials/molecules as represented by graphs of subgraphs and associated target final embeddings.

The MLP function 235 may be trained to process vector data through a training process through which the parameters of the MLP function 235 are updated and optimized. The MLP function 235 is trained based on input vector data and minimization of a loss function that quantifies the difference between a predicted output and a target output, each representing predicted material properties according to the processing function. The training data set upon which the MLP function 235 is trained can include a dataset of vectors (each representing a material/molecule) and the associated target output.

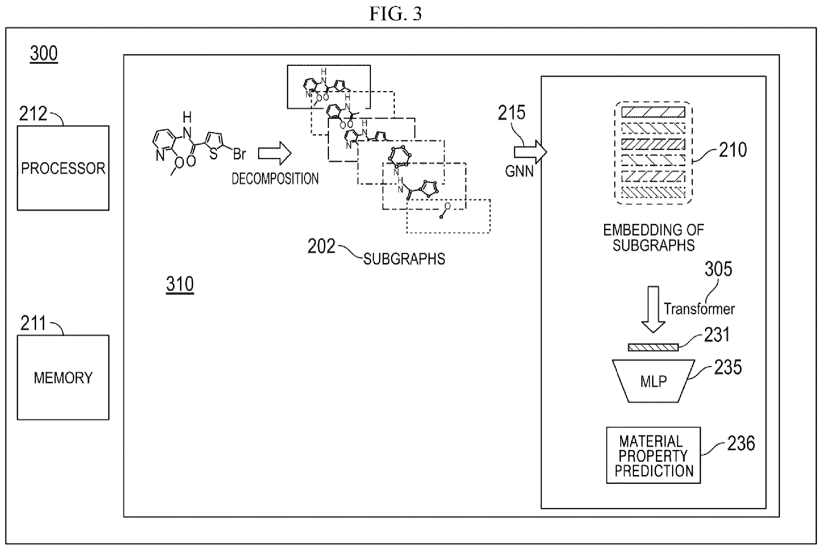

FIG. 3 is a block diagram depicting a computer device 300 for material property prediction, including another configuration of a material subgraph modeling circuit 310 implementing subgraph modeling, according to some embodiments of the present disclosure.

The computer device 300 and material subgraph modeling circuit 310 may have a substantially similar configuration and/or functionality as described with respect to FIG. 2. For purposes of brevity, the differentiating components and/or functionality of the material subgraph modeling circuit 310, according to some embodiments, are described in detail in reference to FIG. 3.

The material subgraph modeling circuit 310 may be configured to implement a transformer 305 to learn the relationship between subgraphs 202. For example, the material subgraph modeling circuit 310 may utilize the transformer 305 to process the subgraph embeddings 210, and generate updated embeddings from the subgraph embeddings 210 (e.g., without generating the graph of subgraphs 220).

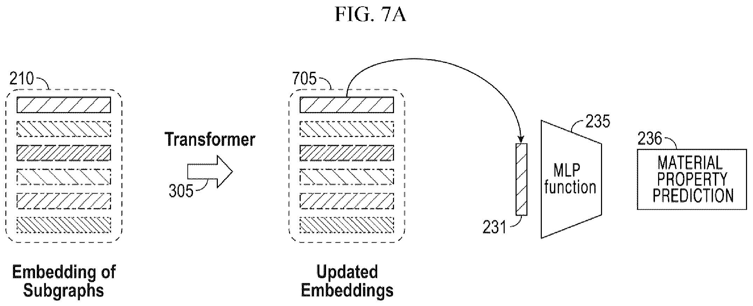

FIG. 7A depicts an example of a process for generating updated embeddings 705 implemented by the material subgraph modeling circuit 310, according to some embodiments of the present disclosure. The transformer 305 may be configured to model a non-linear interdependency between tokens, and thus can be leveraged to model the interdependency between the subgraphs that are represented by the subgraph embeddings 210. The transformer 305 may generate the updated embeddings 705 by passing the subgraph embeddings 210 through a series of layers where each embedding can be refined based on its context within the sequence, generating contextually aware embeddings that may capture the relationships between subgraphs, thereby modeling the relationships between the molecular substructures. The transformer 305 may be trained to process embeddings through a training process through which parameters of the transformer 305 may be optimized through minimizing a loss function representing the difference between predicted embeddings and target embeddings. The training data set upon which the transformer 305 is trained can include a dataset of materials/molecules, each represented by subgraph embeddings and associated target updated embeddings.

The updated embeddings 705 (for the multiple subgraphs) may be aggregated, which can be generated as a single vector 231 (representing the aggregation of the updated embeddings 705). In some embodiments, the result (e.g., calculations of the last layer of the transformer 305) for the updated embeddings 705 may be utilized to generate the material property prediction 236 as the output. The vector 231 may be passed to the MLP function 235, which processes the result from the updated embeddings 705 for further processing tasks, such as classification, regression, and/or the like, and outputs the material property prediction 236.

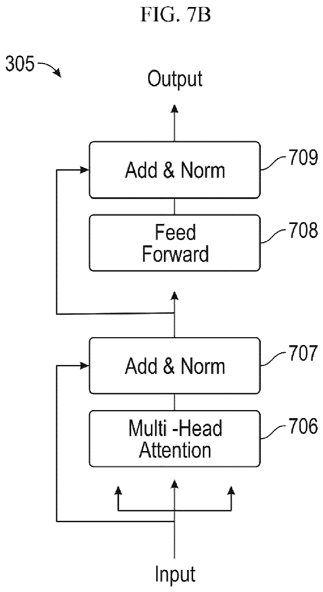

FIG. 7B depicts an example of circuitry in the transformer 305 that may be utilized to implement the transformer functionality. The circuitry of the transformer 305 may include: multi-head attention circuitry 706 (represented as Multi-Head Attention) that may be configured to execute multiple attention mechanisms in parallel to process information from an input sequence and then concatenated and/or linearly transform the dependent attention outputs into an expected dimension; add and norm circuitry 707, 709 (represented as Add & Norm) that may be configured to perform combined operations where the output of a layer is added to its original input (e.g., add) and then followed by a layer normalization step ("Norm"); and feedforward circuitry 708 (represented as Feed Forward) which may be configured to pass information in a direction. Thus, the material subgraph modeling circuit 310 may leverage the relationship modeling capabilities of the transformer 305 in a manner that may achieve improved accuracy in the material property prediction 236 by maintaining critical interactions.

FIG. 8 is a block diagram of an electronic device in a network environment, according to some embodiments of the present disclosure.

Referring to FIG. 8, an electronic device 801 in a network environment 800 may communicate with an external electronic device 802 via a first network 898 (e.g., a short-range wireless communication network), or with an external electronic device 804 or a server 808 via a second network 899 (e.g., a long-range wireless communication network). The electronic device 801 may communicate with the external electronic device 804 via the server 808. The electronic device 801 may include a processor 820, a memory 830, an input device 850, a sound output device 855, a display device 860, an audio module 870, a sensor module 876, an interface 877, a haptic module 879, a camera module 880, a power management module 888, a battery 889, a communication module 890, a subscriber identification module (SIM) 896, and/or an antenna module 897. In one embodiment, at least one of the components (e.g., the display device 860 or the camera module 880) may be omitted from the electronic device 801, or one or more other components may be added to the electronic device 801. Some of the components may be implemented as a single integrated circuit (IC). For example, the sensor module 876 (e.g., a fingerprint sensor, an iris sensor, or an illuminance sensor) may be embedded in the display device 860.

The processor 820 may execute software (e.g., a program 840) to control at least one other component (e.g., a hardware or a software component) of the electronic device 801 coupled to the processor 820, and may perform various data processing or computations.

As at least part of the data processing or computations, the processor 820 may load a command or data received from another component (e.g., the sensor module 876 or the communication module 890) in volatile memory 832, may process the command or the data stored in the volatile memory 832, and may store resulting data in non-volatile memory 834. The processor 820 may include a main processor 821 (e.g., a central processing unit or an application processor (AP)), and an auxiliary processor 823 (e.g., a graphics processing unit (GPU), an image signal processor (ISP), a sensor hub processor, or a communication processor (CP)) that is operable independently from, or in conjunction with, the main processor 821. Additionally or alternatively, the auxiliary processor 823 may be adapted to consume less power than the main processor 821, or to execute a particular function. The auxiliary processor 823 may be implemented as being separate from, or a part of, the main processor 821.

The auxiliary processor 823 may control at least some of the functions or states related to at least one component (e.g., the display device 860, the sensor module 876, or the communication module 890), as opposed to the main processor 821 while the main processor 821 is in an inactive (e.g., sleep) state, or together with the main processor 821 while the main processor 821 is in an active state (e.g., executing an application). The auxiliary processor 823 (e.g., an image signal processor or a communication processor) may be implemented as part of another component (e.g., the camera module 880 or the communication module 890) functionally related to the auxiliary processor 823.

The memory 830 may store various data used by at least one component (e.g., the processor 820 or the sensor module 876) of the electronic device 801. The various data may include, for example, software (e.g., the program 840) and input data or output data for a command related thereto. The memory 830 may include the volatile memory 832 or the non-volatile memory 834. The non-volatile memory 834 may include an internal memory 836 and/or an external memory 838.

The program 840 may be stored in the memory 830 as software, and may include, for example, an operating system (OS) 842, middleware 844, or an application 846.

The input device 850 may receive a command or data to be used by another component (e.g., the processor 820) of the electronic device 801, from the outside (e.g., a user) of the electronic device 801. The input device 850 may include, for example, a microphone, a mouse, or a keyboard.

The sound output device 855 may output sound signals to the outside of the electronic device 801. The sound output device 855 may include, for example, a speaker or a receiver. The speaker may be used for general purposes, such as playing multimedia or recording, and the receiver may be used for receiving an incoming call. The receiver may be implemented as separate from, or as a part of, the speaker.

The display device 860 may visually provide information to the outside (e.g., to a user) of the electronic device 801. The display device 860 may include, for example, a display, a hologram device, or a projector, and may include control circuitry to control a corresponding one of the display, hologram device, and projector. The display device 860 may include touch circuitry adapted to detect a touch, or may include sensor circuitry (e.g., a pressure sensor) adapted to measure the intensity of force incurred by the touch.

The audio module 870 may convert a sound into an electrical signal and vice versa. The audio module 870 may obtain the sound via the input device 850 or may output the sound via the sound output device 855 or a headphone of an external electronic device 802 directly (e.g., wired) or wirelessly coupled to the electronic device 801.

The sensor module 876 may detect an operational state (e.g., power or temperature) of the electronic device 801, or an environmental state (e.g., a state of a user) external to the electronic device 801. The sensor module 876 may then generate an electrical signal or data value corresponding to the detected state. The sensor module 876 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, and/or an illuminance sensor.

The interface 877 may support one or more specified protocols to be used for the electronic device 801 to be coupled to the external electronic device 802 directly (e.g., wired) or wirelessly. The interface 877 may include, for example, a high-definition multimedia interface (HDMI), a universal serial bus (USB) interface, a secure digital (SD) card interface, or an audio interface.

A connecting terminal 878 may include a connector via which the electronic device 801 may be physically connected to the external electronic device 802. The connecting terminal 878 may include, for example, an HDMI connector, a USB connector, an SD card connector, or an audio connector (e.g., a headphone connector).

The haptic module 879 may convert an electrical signal into a mechanical stimulus (e.g., a vibration or a movement) or an electrical stimulus, which may be recognized by a user via tactile sensation or kinesthetic sensation. The haptic module 879 may include, for example, a motor, a piezoelectric element, or an electrical stimulator.

The camera module 880 may capture a still image or moving images. The camera module 880 may include one or more lenses, image sensors, image signal processors, or flashes. The power management module 888 may manage power that is supplied to the electronic device 801. The power management module 888 may be implemented as at least part of, for example, a power management integrated circuit (PMIC).

The battery 889 may supply power to at least one component of the electronic device 801. The battery 889 may include, for example, a primary cell that is not rechargeable, a secondary cell that is rechargeable, or a fuel cell.

The communication module 890 may support establishing a direct (e.g., wired) communication channel or a wireless communication channel between the electronic device 801 and the external electronic device (e.g., the external electronic device 802, the external electronic device 804, or the server 808), and may support performing communication via the established communication channel. The communication module 890 may include one or more communication processors that are operable independently from the processor 820 (e.g., the AP), and may support a direct (e.g., wired) communication or a wireless communication. The communication module 890 may include a wireless communication module 892 (e.g., a cellular communication module, a short-range wireless communication module, or a global navigation satellite system (GNSS) communication module) or a wired communication module 894 (e.g., a local area network (LAN) communication module or a power line communication (PLC) module). A corresponding one of these communication modules may communicate with the external electronic device via the first network 898 (e.g., a short-range communication network, such as BLUETOOTHTM, wireless-fidelity (Wi-Fi) direct, or a standard of the Infrared Data Association (IrDA)), or via the second network 899 (e.g., a long-range communication network, such as a cellular network, the Internet, or a computer network (e.g., LAN or wide area network (WAN)). These various types of communication modules may be implemented as a single component (e.g., a single IC), or may be implemented as multiple components (e.g., multiple ICs) that are separate from each other. The wireless communication module 892 may identify and authenticate the electronic device 801 in a communication network, such as the first network 898 or the second network 899, using subscriber information (e.g., international mobile subscriber identity (IMSI)) stored in the subscriber identification module 896.

The antenna module 897 may transmit or receive a signal or power to or from the outside (e.g., the external electronic device) of the electronic device 801. The antenna module 897 may include one or more antennas. The communication module 890 (e.g., the wireless communication module 892) may select at least one of the one or more antennas appropriate for a communication scheme used in the communication network, such as the first network 898 or the second network 899. The signal or the power may then be transmitted or received between the communication module 890 and the external electronic device via the selected at least one antenna.

Commands or data may be transmitted or received between the electronic device 801 and the external electronic device 804 via the server 808 coupled to the second network 899. Each of the external electronic devices 802 and 804 may be a device of a same type as, or a different type, from the electronic device 801. All or some of operations to be executed at the electronic device 801 may be executed at one or more of the external electronic devices 802, 804, or the server 808. For example, if the electronic device 801 should perform a function or a service automatically, or in response to a request from a user or another device, the electronic device 801, instead of, or in addition to, executing the function or the service, may request the one or more external electronic devices to perform at least part of the function or the service. The one or more external electronic devices receiving the request may perform the at least part of the function or the service requested, or an additional function or an additional service related to the request and transfer an outcome of the performing to the electronic device 801. The electronic device 801 may provide the outcome, with or without further processing of the outcome, as at least part of a reply to the request. To that end, cloud computing, distributed computing, or client-server computing technology may be used, for example.

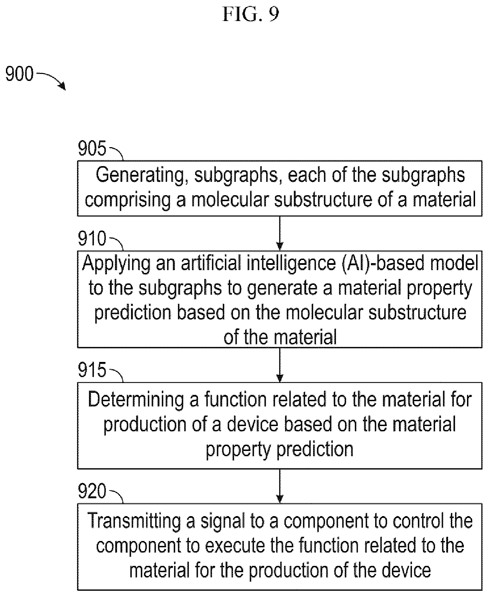

FIG. 9 is a flow chart depicting example operations of a method 900 for utilizing AI-based material subgraph models and/or enhanced material property predictions, according to some embodiments of the present disclosure. For example, FIG. 9 illustrates various operations in a method 900 for material subgraph models and/or enhanced material property predictions, according to some embodiments. Although FIG. 9 illustrates various operations in a method according to some embodiments, embodiments according to the present disclosure are not limited thereto, and according to various embodiments, the method may include additional operations or fewer operations without departing from the scope of embodiments according to the present disclosure.

Referring to FIG. 9 the method 900 may include one or more of the following operations. A processor 212 (see FIG. 2) may generate subgraphs, where each of the subgraphs may include a molecular substructure of a material (operation 905). A processor 212 may apply an AI-based model to the subgraphs to generate a material property prediction based on the molecular substructure of the material (operation 910). The processor 212 may determine a function related to the material for production of a device based on the material property prediction (operation 915). The function may be related to design, fabrication and/or synthesis, testing and/or validation, and/or utilization (e.g., during manufacturing of an OLED device) of the material. A processor 212 may transmit a signal to a component, for example system 108 (see FIG. 1), to control the component to execute the function related to the material for the production of the device based on the determining (operation 920).

For example, according to some embodiments, transmitting the signal may include transmitting a control signal to a component operating as part of a production line that may physically retrieve, handle, and/or process the synthesis of a material (e.g., in an automated fashion, without manual human intervention) to be utilized as part of a manufacturing process of a device. Additionally, according to some embodiments, transmitting the signal may include transmitting a signal to computer system including a display device operating as a manufacturing component as part of a production line or production facility to display a result of the determination thereon.

Accordingly, aspects of some embodiments of the present disclosure may provide systems and/or functions related to AI-based material subgraph models, including the decomposition of the molecular structure of materials, generating graphs of subgraphs, and implementing subgraph modeling in a manner that may scale AI-based models to large and/or complex molecules and enhances the material property predictions. Thus, the disclosed embodiments may improve the overall performance of display related products by utilizing materials deemed most suitable and/or efficient.

Embodiments of the subject matter and the operations described in this specification may be implemented in digital electronic circuitry, or in computer software, firmware, or hardware, including the structures disclosed in this specification and their structural equivalents, or in combinations of one or more of them. Embodiments of the subject matter described in this specification may be implemented as one or more computer programs, i.e., one or more modules of computer-program instructions, encoded on computer-storage medium for execution by, or to control the operation of data-processing apparatus. Alternatively or additionally, the program instructions can be encoded on an artificially-generated propagated signal, e.g., a machine-generated electrical, optical, or electromagnetic signal, which is generated to encode information for transmission to suitable receiver apparatus for execution by a data processing apparatus. A computer-storage medium can be, or be included in, a computer-readable storage device, a computer-readable storage substrate, a random or serial-access memory array or device, or a combination thereof. Moreover, while a computer-storage medium is not a propagated signal, a computer-storage medium may be a source or destination of computer-program instructions encoded in an artificially-generated propagated signal. The computer-storage medium can also be, or be included in, one or more separate physical components or media (e.g., multiple CDs, disks, or other storage devices). Additionally, the operations described in this specification may be implemented as operations performed by a data-processing apparatus on data stored on one or more computer-readable storage devices or received from other sources.

While this specification may contain many specific implementation details, the implementation details should not be construed as limitations on the scope of any claimed subject matter, but rather be construed as descriptions of features specific to particular embodiments. Certain features that are described in this specification in the context of separate embodiments may also be implemented in combination in a single embodiment. Conversely, various features that are described in the context of a single embodiment may also be implemented in multiple embodiments separately or in any suitable subcombination. Moreover, although features may be described above as acting in certain combinations and even initially claimed as such, one or more features from a claimed combination may in some cases be excised from the combination, and the claimed combination may be directed to a subcombination or variation of a subcombination.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system components in the embodiments described above should not be understood as requiring such separation in all embodiments, and it should be understood that the described program components and systems can generally be integrated together in a single software product or packaged into multiple software products.

Thus, particular embodiments of the subject matter have been described herein. Other embodiments are within the scope of the following claims. In some cases, the actions set forth in the claims may be performed in a different order and still achieve desirable results. Additionally, the processes depicted in the accompanying figures do not necessarily require the particular order shown, or sequential order, to achieve desirable results. In certain implementations, multitasking and parallel processing may be advantageous.

As will be recognized by those skilled in the art, the innovative concepts described herein may be modified and varied over a wide range of applications. Accordingly, the scope of claimed subject matter should not be limited to any of the specific example teachings discussed above, but is instead defined by the following claims. Further Embodiments are set out in the following clauses:
Clause 1. A method comprising:
   generating, by a processor, subgraphs, each of the subgraphs comprising a molecular substructure of a material;
   applying, by the processor, an artificial intelligence (AI)-based model to the subgraphs to generate a material property prediction based on the molecular substructure of the material;
   determining, by the processor, a function related to the material for production of a target device based on the material property prediction; and
   transmitting, by the processor, a signal to a component to control the component to execute the function related to the material for the production of the target device.
Clause 2. The method of clause 1, further comprising decomposing a graph of a molecular structure of the material into the molecular substructures.
Clause 3. The method of clause 2, wherein the decomposing comprises Breaking Retrosynthetically Interesting Chemical bonds (BRIC) decomposition.
Clause 4. The method of any preceding clause, further comprising generating embeddings of the subgraphs based on the subgraphs.
Clause 5. The method of clause 4, wherein the generating the embeddings of the subgraphs comprises processing the subgraphs by a graph neural network.
Clause 6. The method of clause 4 or clause 5, further comprising generating a graph of subgraphs based on the embeddings of the subgraphs.
Clause 7. The method of clause 6, wherein the graph of subgraphs comprises nodes and edges.
Clause 8. The method of clause 7, wherein each of the nodes correspond to one of the subgraphs, and a value for each of the nodes correspond to one of the embeddings of the subgraphs. Clause 9. The method of clause 8, wherein each of the edges represent a relationship between the subgraphs.
Clause 10. The method of any one of clauses 6 - 9, further comprising generating updated node embeddings based on the graph of subgraphs.
Clause 11. The method of clause 10, wherein the generating the updated node embeddings comprises processing the graph of subgraphs by a graph neural network.
Clause 12. The method of clause 9 or clause 10, wherein the AI-based model analyzes the graph of subgraphs and models the relationships between the subgraphs.
Clause 13. The method of any preceding clause, wherein the target device comprises an organic light-emitting diode (OLED) display device.
Clause 14. The method of any preceding clause, wherein the material property prediction is based one or more of: a physical property of the material, a chemical property of the material, mechanical property of the material, or an optical property of the material.
Clause 15. A device comprising:
   one or more processors that are configured to perform:
   generating subgraphs, each of the subgraphs comprising a molecular substructure of a material;
   applying an artificial intelligence (AI)-based model to the subgraphs to generate a material property prediction based on the molecular substructure of the material;
   determining a function related to the material for production of a target device based on the material property prediction; and
   transmitting a signal to a component to control the component to execute the function related to the material for the production of the target device.
Clause 16. The device of clause 15, wherein the one or more processors are further configured to perform decomposing a graph of a molecular structure of the material into the molecular substructures.
Clause 17. The device of clause 15 or clause 16, wherein the one or more processors are further configured to perform generating embeddings of the subgraphs based on the subgraphs.
Clause 18. The device of clause 17, wherein the one or more processors are further configured to perform generating a graph of subgraphs based on the embeddings of the subgraphs.
Clause 19. The device of clause 18, wherein the one or more processors are further configured to perform analyzing the graph of subgraphs using the AI-based model and modeling relationships between the subgraphs.
Clause 20. A system comprising:
   a processing circuit; and
   a memory storing instructions, which, based on being executed by the processing circuit, cause the processing circuit to perform:
      generating subgraphs, each of the subgraphs comprising a molecular substructure of a material;
      applying an artificial intelligence (AI)-based model to the subgraphs to generate a material property prediction based on the molecular substructure of the material;
      determining a function related to the material for production of a target device based on the material property prediction; and
      transmitting a signal to a component to control the component to execute the function related to the material for the production of the target device.

## Claims

1. A method comprising:
generating, by a processor (212), subgraphs (202), each of the subgraphs (202) comprising a molecular substructure of a material (201);
applying, by the processor, an artificial intelligence (AI)-based model to the subgraphs to generate a material property prediction (236) based on the molecular substructure of the material (201);
determining, by the processor (212), a function related to the material for production of a target device based on the material property prediction; and
transmitting, by the processor (212), a signal to a component (102) to control the component to execute the function related to the material (201) for the production of the target device.

2. The method of claim 1, further comprising decomposing a graph of a molecular structure of the material into the molecular substructures.

3. The method of claim 2, wherein the decomposing comprises Breaking Retrosynthetically Interesting Chemical bonds (BRIC) decomposition.

4. The method of any preceding claim, further comprising generating embeddings (210) of the subgraphs (202) based on the subgraphs (202).

5. The method of claim 4, wherein the generating the embeddings (210) of the subgraphs (202) comprises processing the subgraphs (202) by a graph neural network (215).

6. The method of claim 4 or claim 5, further comprising generating a graph of subgraphs (220) based on the embeddings (210) of the subgraphs (202).

7. The method of claim 6, wherein the graph of subgraphs (220) comprises nodes (221) and edges (222).

8. The method of claim 7, wherein each of the nodes (221) correspond to one of the subgraphs (202), and a value for each of the nodes (221) correspond to one of the embeddings (210) of the subgraphs (202).

9. The method of claim 8, wherein each of the edges (222) represent a relationship between the subgraphs (202).

10. The method of any one of claims 6 - 9, further comprising generating updated node embeddings (605) based on the graph of subgraphs;
wherein, optionally, the generating the updated node embeddings (605) comprises processing the graph of subgraphs (220) by a graph neural network (230).

11. The method of claim 10, wherein the AI-based model analyzes the graph of subgraphs (220) and models the relationships between the subgraphs (220).

12. The method of any preceding claim, wherein the target device comprises an organic light-emitting diode (OLED) display device.

13. The method of any preceding claim, wherein the material property prediction (236) is based one or more of: a physical property of the material, a chemical property of the material, mechanical property of the material, or an optical property of the material.

14. A device comprising:
one or more processors (212) that are configured to perform a method according to any preceding claim.

15. A system (102) comprising a device according to claim 14, and a memory (211) storing instructions, which, based on being executed by the processing circuit, cause the processing circuit to perform the method according to any one of claims 1 - 13.
